# EUROPEAN PATENT SPECIFICATION

(11) **EP 1 011 664 B1**
(45) Date of publication and mention of the grant of the patent: **24.11.2004**
(21) Application number: 98949319.2
(22) Date of filing: 04.09.1998
(51) Int. Cl.: A61K 31/70, A61P 31/04

(54) **8a-AZALIDES AS VETERINARY ANTIMICROBIAL AGENTS**
8a-AZALIDEN ALS ANTIMIKROBIELLE AGENTEN
8a-AZALIDES UTILISES COMME AGENTS ANTIMICROBIENS VETERINAIRES

(30) Priority: 10.09.1997 US 58329 P; 20.03.1998 GB 9806029
(43) Date of publication of application: 28.06.2000
(73) Proprietor: MERCK & CO., INC., Rahway, New Jersey 07065 (US)
(72) Inventor: KROPP, Helmut, Rahway, NJ 07065 (US); FARRINGTON, Daniel, O., Rahway, NJ 07065 (US); CLARK, Jeffrey, N., Rahway, NJ 07065 (US); RATCLIFFE, Ronald, W., Rahway, NJ 07065 (US); WILKENING, Robert, D., Rahway, NJ 07065 (US)
(74) Representative: Horgan, James Michael Frederic
(86) International application number: PCT/US1998/018573
(87) International publication number: WO 1999/012542

(56) References cited:
- EP-A- 0 316 163
- EP-A- 0 508 699
- EP-A- 0 549 040
- US-A- 4 956 294
- US-A- 5 189 159
- US-A- 5 202 434
- US-A- 5 281 518
- GILL, CHARLES J. ET AL: "In vivo evaluation of three acid-stable azalide compounds, L-701,677, L-708,299 and L-708,365 compared to erythromycin, azithromycin and clarithromycin" J. ANTIBIOT. (1995), 48(10), 1141-7 , XP000982810
- K SHANKARAN ET AL: "Preparation and Activities of 4-Epi and 4-deoxy-4-Amino Analogs Derived From 9-Deoxo-8a-Aza-8a-Homoerythromycin A" BIOORGANIC & MEDICINAL CHEMISTRY LETTERS,GB,OXFORD, vol. 4, no. 9, 1994, pages 1111-1116, XP002118137 ISSN: 0960-894X
- DATABASE WPIDS ON STN, DERWENT INFORMATION LTD., No. 92-341964, HECK J.V. et al., "New 9-De oxo-8A-aza-8A-Homo-Erythromycin A Derivatives - Useful as Antibiotics and Intermediates for Synthesis of Other Macrolide Antibiotics", XP002915137; & EP,A,508 699.

## Description

### SUMMARY OF THE INVENTION

The present invention provides methods for the treatment or prevention of bacterial respiratory or enteric infections in livestock animals.

### BACKGROUND OF THE INVENTION

The morbidity and mortality associated with bacterial respiratory and enteric infections in livestock represent a major economic loss for the animal husbandry industry. In cattle, especially in younger animals, stress as a result of weaning, transportation, dehydration, alteration or deprivation of diet can cause the animals to become highly susceptible to bacterial respiratory infection, especially if the animals are housed in crowded or poorly ventilated quarters. The principal causative bacterial pathogens of bovine respiratory infections are *Pasteurella haemolytica, P. multocida, Haemophilus somnus* and *Mycoplasma* spp. In pigs respiratory infections caused by *Pasteurella multocida* or *Actinobacillus pleuropneumoniae*, and *Mycoplasma* spp. are associated with considerable losses in some herds. The most common causative organisms for enteric diseases in cattle and swine are *Escherichia coli, Treponema hyodysenteriae* and *Salmonella* spp.

The current therapeutic antimicrobial products against respiratory and enteric infections in livestock include a diverse group of older products effective against a broad spectrum of infectious agents, mostly notably among this group are the tetracyclines; and a group of recently introduced products indicated primarily for treatment of bovine respiratory disease, such as quinolones (danofloxacin, enrofloxacin), cephalosporins (cefquinome, ceftiofur), macrolide (tilmicosin), and florfenicol. Resistance to the older antimicrobial agents has developed in the field. Although resistance to the newer products is not yet a problem, it is known that excessive usage favors the emergence of resistance over time, but increasing the numbers of drug families, and thereby mechanisms of action, in use may decrease the probability of resistance development to any individual compound. Thus, there exists a continuing need to discover antimicrobial compounds that are suitable for use in veterinary medicine; preferably, such compounds will belong to a different chemotype from the antimicrobial agents currently in use in animal or human medicine. Other desirable characteristics of a novel antimicrobial product for veterinary use include high potency against target organisms, high target tissue concentration, and long tissue and plasma half-life.

8a-Azalides are antibiotics characterized by a 15-membered lactone ring containing a ring nitrogen atom. A group of 8a-azalides are disclosed in European Patent Application 508,699 as having antibacterial spectrum similar to that of erythromycin, and as being active in vitro against gram positive and gram negative bacteria, including *E*. *coli*, and *H. influenzae*. However, EP 508,699 does not disclose the use of 8a-azalides for the treatment and prevention of bacterial respiratory or enteric infections in livestock animals. Furthermore, there is no suggestion that the 8a-azalides have antibacterial activity against the common causative organisms of cattle and swine bacterial respiratory and enteric infections.

### DETAILED DESCRIPTION OF THE INVENTION

The present invention provides the use of an 8a-azalide for the manufacture of a medicament for the treatment or prevention of a bacterial bovine or swine respiratory infection caused by a Pasteurella spp., an Actino bacillus spp., Haemophilus somnus or Mycoplasma spp.

There is also provided the use of an 8a-azalide for the manufacture of a medicament for the treatment or prevention of a bacterial bovine or swine enteric infection caused by, for example, E.coli, Treponema hyodysenteriae or a Salmonella spp.

In a preferred embodiment the 8a-azalide has the formula I: 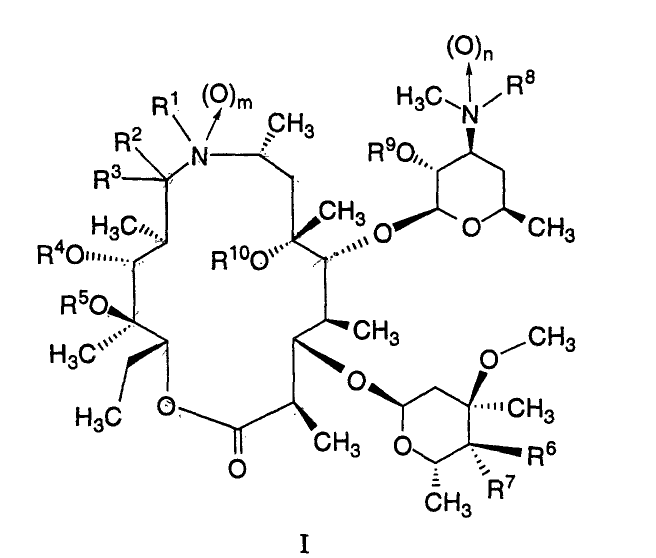 or a pharmaceutically acceptable salt thereof, or a pharmaceutically acceptable metal complexes thereof, and said metal complex is taken from the group consisting of copper, zinc, cobalt, nickel and cadmium; where
- R¹: is hydrogen;
hydroxy;
C₁₋₄ alkoxy;
formyl;

C₁₋₁₀ alkylcarbonyl, C₁₋₁₀ alkoxycarbonyl, aryloxycarbonyl, C₁₋₁₀ aralkoxycarbonyl, C₁₋₁₀ alkylsulfonyl, or arylsulfonyl wherein said C₁₋₁₀ alkyl group is substituted by 1-3 halo (F,Cl,Br), hydroxy, amino, C₁₋₅ acylamino or C₁₋₄ alkyl groups; or
unsubstituted or substituted C₁₋₁₀ alkyl, C₂₋₁₀ alkenyl or C₂₋₁₀ alkynyl wherein said alkyl chain, if more than 2 carbons in length, can have inserted therein 1 to 2 of oxa, thia or aza of the formula -NR-where R is hydrogen or C₁₋₃ alkyl, and wherein said substituents are independently 1-3 of
(a) aryl or heteroaryl optionally substituted by 1-3 halo (F, Cl, Br, I), C₁₋₄ alkyl, C₁₋₃ alkoxy, amino, C₁₋₄ alkylamino, di(C₁₋₄ alkyl)amino or hydroxy,
(b) heterocyclyl unsubstituted or substituted by hydroxy, amino, C₁₋₄ alkylamino, di(C₁₋₄ alkyl) amino, C₁₋₄ alkylcarbonyloxy or C₁₋₄ alkylcarbonylamino,
(c) halo (F,Cl,Br or I),
(d) hydroxy non-acylated or acylated by a group R^{a}C(=O) or R^{b} S(O)2 wherein R^{a} is hydrogen, C₁₋₆ alkyl, aryl, heteroaryl, aralkyl, or heteroaralkyl and R^{b} is C₁₋₆ alkyl or aryl,
(e) C₁₋₁₀ alkoxy,
(f) aryloxy or heteroaryloxy unsubstituted or substituted by 1-3 halo, hydroxy, amino or C₁₋₄ alkyl groups,
(g) amino or C₁₋₁₀ alkylamino non-acylated or acylated by a group R^{a}C(=O), R^{a}OC(=O), or R^{b}SO₂, wherein R^{a} and R^{b} are as defined above;
(h) di(C₁₋₁₀ alkyl)amino,
(i) arylamino, heteroarylamino, aralkylamino or heteroarylalkylamino wherein said aryl or heteroaryl group is unsubstituted or substituted by 1-3 halo, hydroxy, amino or C₁-C₄ alkyl groups,
(j) mercapto,
(k) C₁₋₁₀ alkylthio, alkylsulfinyl or alkylsulfonyl, arylthio, arylsulfinyl or arylsulfonyl wherein said aryl group is unsubstituted or substituted by 1-3 halo, hydroxy, amino or C₁₋₄ alkyl groups,
(l) formyl,
(m) C₁₋₁₀ alkylcarbonyl,
(n) arylcarbonyl, heteroarylcarbonyl, aralkylcarbonyl or heteroarylalkylcarbonyl wherein said aryl or heteroaryl group is unsubstituted or substituted by 1-3 halo, hydroxy, amino or C-₄ alkyl groups,
(o) carboxy,
(p) C₁₋₁₀ alkoxycarbonyl,
(q) aryloxycarbonyl, heteroaryloxycarbonyl, aralkoxycarbonyl or heteroarylalkoxycarbonyl wherein said aryl or heteroaryl group is unsubstituted or substituted by 1-3 halo, hydroxy, amino or C₁₋₄ alkyl groups,
(r) carbamoyl or sulfamoyl wherein the N-atom is unsubstituted or substituted by 1-2 C₁₋₆ alkyl groups or by a C₄₋₆ alkylene chain,
(s) cyano,
(t) isonitrilo
(u) nitro,
(v) azido,
(w) iminomethyl unsubstituted or substituted on nitrogen or carbon with C₁₋₁₀ alkyl,
(x) oxo or
(y) thiono;
R² and R³ are independently
hydrogen,
C₁₋₁₀ alkyl,
aryl; or
R² and R³ together are oxo or thiono;
R⁴ and R⁵ are independently hydrogen or alkylcarbonyl; or
R⁴ and R⁵ are together carbonyl; or
R⁴ and R¹ together are C₁-C₃ alkylene unsubstituted or substituted by an oxo group;
R⁶ and R⁷ are both hydrogen, or
one of R⁶ and R⁷ is hydrogen and the other is
hydroxy,
an acyloxy derivative taken from the group consisting of formyloxy, C₁₋₁₀ alkylcarbonyloxy, arylcarbonyloxy and aralkylcarbonyloxy, or
-NHR¹² wherein R¹² is hydrogen, arylsulfonyl or heteroarylsulfonyl unsubstituted or substituted by 1-3 halo or C₁₋₃ alkyl groups, alkylsulfonyl or - C(=O)-X-A-R¹³ where X is a connecting bond, O or NH, A is a connecting bond or C₁-C₃ alkylene, R¹³ is hydrogen, C₁-C₁₀ alkyl, aryl, aralkyl, heteroaryl, heterocyclyl, or C₃-C₇ cycloalkyl, any of which R¹³ groups other than hydrogen can be substituted by one or more of halogen, hydroxyl, C₁-C₃ alkoxy, cyano, isonitrilo, nitro, amino, mono- or di- (C₁-C₃) alkylamino, mercapto, C₁-C₃ alkylthio, C₁-C₃ alkylsulfinyl, C₁-C₃ alkylsulfonyl, arylthio, arylsulfinyl, sulfamoyl, arylsulfonyl, carboxy, carbamoyl, C₁-C₃ alkylcarbonyl, or C₁-C₃ alkoxycarbonyl; or
R⁶ and R⁷ are together oxo, hydroxyimino, alkoxyimino, aralkoxyimino or aminoimino;
- R⁸: is methyl,
aralkoxycarbonyl, or
arylsulfonyl;
- R⁹: is
hydrogen,
formyl,
C₁₋₁₀ alkylcarbonyl,
C₁₋₁₀ alkoxycarbonyl, or
arylalkoxycarbonyl;
- R¹⁰: is hydrogen; or
R¹⁰ and R¹ together are C₁-C₃ alkylene unsubstituted or substituted by an oxo group;
m and n are independently zero or one.

More preferably, the 8a-azalide has the formula I wherein n and m are zero;
R¹ is hydrogen, C₁₋₁₀ alkyl, C₂₋₁₀ alkenyl, C₂₋₁₀ alkynyl or arylsulfonyl, wherein said alkyl and alkenyl are optionally substituted with halo, hydroxy, cyano, C₁₋₁₀ alkoxycarbonyl, amino, C₁₋₁₀ alkylamino, di(C₁₋₁₀ alkylamino), aryl or aralkoxycarbonyl;
R², R³, R⁴, R⁵, R⁹ and R¹⁰ are each hydrogen;
one of R6 and R7 is hydrogen and the other is selected from hydroxyl, C₁₋ ₁₀ alkyl carbonyloxy, aralkylcarbonyloxy, amino, amino substituted by C₁₋₁₀ alkylcarbonyl, arylcarbonyl, aryl C₁₋₁₀ alkylcarbonyl, C₁₋₁₀ alkoxycarbonyl, aryl C₁₋₁₀ alkoxycarbonyl, heteroarylcarbonyl, heteroarylalkylcarbonyl or arylsulfonyl;
R8 is methyl.

Even more preferred are 8a-azalide of the formula I wherein
n and m are zero;
R¹ is methyl, ethyl, propyl, allyl, propargyl, 2-cyanoethyl, 2-hydroxyethyl, 3-hydroxypropyl, 2-methoxycarbonylethyl, 2-benzyloxycarbonylethyl, cyanomethyl, 2-aminoethyl, 2-(dimethylamino)ethyl, 2-fluoroethyl, 2-fluoroallyl, benzyl or oxiranylmethyl;
R², R³, R⁴, R⁵, R⁹ and R¹⁰ are hydrogen;
one of R6 or R7 is hydrogen and the other is hydroxy or amino;
R8 is methyl. The most preferred R¹ groups are methyl, ethyl, propyl, allyl, 2-methoxycarbonylethyl or 2-(dimethylamino)ethyl.

There is also disclosed a method for the treatment or prevention of bovine or swine bacterial respiratory infections wherein the causative organism is selected from a group consisting of *Pasteurella* spp., an *Actinobacillus* spp., *Haemophilus somnus* and *Mycoplasma* spp., which comprises administering to a cattle or swine in need of such treatment or prevention a therapeutically or prophylactically effective amount of an 8a-azalide. More preferably, the 8a-azalide is of formula I; and particularly preferred are 8a-azalides of formula I wherein R¹ is methyl, ethyl, propyl, allyl, propargyl, 2-cyanoethyl, 2-hydroxyethyl, 3-hydroxypropyl, 2-methoxycarbonylethyl, 2-benzyloxycarbonylethyl, cyanomethyl, 2-aminoethyl, 2-(dimethylamino)ethyl, 2-fluoroethyl, 2-fluoroallyl, benzyl or oxiranylmethyl; R², R³, R⁴, R⁵, R⁹ and R¹⁰ are hydrogen;
one of R6 or R7 is hydrogen and the other is hydroxy or amino; R8 is methyl. The most preferred R¹ groups are methyl, ethyl, propyl, allyl, 2-methoxycarbonylethyl or 2-(dimethylamino)ethyl.

There is also disclosed a method for the treatment or prevention of bovine or swine bacterial enteric infections wherein the causative organism is selected from *Escherichia coli, Treponema hyodysenteriae*, and *Salmonella* spp., which comprises administering to a cattle or swine in need of such treatment or prevention a therapeutically or prophylactically effective amount of an 8a-azalide. More preferably the 8a-azalide is of formula I; and particularly preferred are 8a-azalides of formula I wherein R¹ is methyl, ethyl, propyl, allyl, propargyl, 2-cyanoethyl, 2-hydroxyethyl, 3-hydroxypropyl, 2-methoxycarbonylethyl, 2-benzyloxycarbonylethyl, cyanomethyl, 2-aminoethyl, 2-(dimethylamino)ethyl, 2-fluoroethyl, 2-fluoroallyl, benzyl or oxiranylmethyl; R², R³, R⁴, R⁵, R⁹ and R¹⁰ are hydrogen;
one of R6 or R7 is hydrogen and the other is hydroxy or amino;
R8 is methyl. The most preferred R¹ groups are methyl, ethyl, propyl, allyl, 2-methoxycarbonylethyl or 2-(dimethylamino)ethyl.

As used herein "8a-azalide" means a compound having the following core structure in which the asterisks indicate sites for substitution:

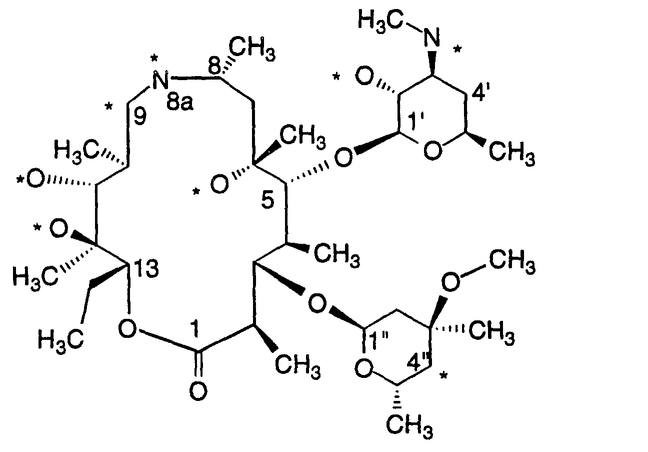

The 8a-azalides are named herein as derivatives of erythromycin A, namely as derivatives of 9-deoxo-8a-aza-8a-homoerythromycin A.

The term "therapeutically or prophylactically effective amount" means that amount of an 8a-azalide that will provide a level of antibacterial activity at the target site of infection that is sufficient to inhibit the bacteria in a manner that allows the host animal to overcome or be protected from the infection.

"Treatment or prevention" means use of 8a-azalide following or prior to manifestation of signs and symptoms suggestive of bacterial infection to allow the host animal to overcome or be protected from the infection.

"Bacterial respiratory or enteric infections" means infections of the respiratory or digestive tract for which the causative organism or the likely causative organism is susceptible to 8a-azalide. Such organisms include, but are not limited to, *Pasteurella* species (e. g. *P. haemolytica, P. multocida*), *Haemophilus somnus, Actinobacillus pleuropneumoniae, Mycoplasma* spp., *E. coli, Treponema hyodysenteriae,* and *Salmonella* spp. (e.g. *S. typhimurium, S. dublin*).

The terms used in defining the variable groups of formula I (e.g. alkyl, aryl, heterocyclyl, substituted, etc) have the same meanings as those provided in EP 508,699.

The term "pharmaceutically acceptable salts" refers to salts prepared from pharmaceutically acceptable non-toxic acids including inorganic acids and organic acids. Such acids include acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid, and the like. Particularly preferred are citric, hydrobromic, hydrochloric, maleic, phosphoric, sulfuric, and tartaric acids.

8a-Azalides are either known compounds, such as those disclosed in European Application 508,699, or they may be prepared using known methods from readily available starting materials. Representative 8a-azalides are as follows:
9-Deoxo-8a-aza-8a-homoerythromycin A;
9-Deoxo-8a-aza-8a-methyl-8a-homoerythromycin A;
9-Deoxo-8a-aza-8a-ethyl-8a-homoerythromycin A;
8a-(3-phenylpropyl)-8a-aza-9-deoxo-8a-homoerythromycin A;
9-Deoxo-8a-aza-8a-allyl-8a-homoerythromycin A;
9-Deoxo-8a-aza-8a-(prop-1-yl)-8a-homoerythromycin A;
9-Deoxo-8a-aza-8a-(prop-1-yloxy)-8a-homoerythromycin A;
9-Deoxo-8a-aza-8a-(2-oxoeth-1-yl)-8a-homoerythromycin A;
9-Deoxo-8a-aza-8a-(2-hydroxyeth-1-yl)-8a-homoerythromycin A;
9-Deoxo-8a-aza-8a-((2,3-epoxy)prop-1-yl)-8a-homoerythromycin A;
9-Deoxo-8a-aza-8a-(11-azetidinyl)-2-ethl-yl)-8a-homoerythromycin A;
9-Deoxo-8a-aza-8a-((1-pyrrolidinyl)-2-eth-1-yl)-8a-homoerthromycin A;
9-Deoxo-8a-aza-8a-((N-piperidinyl)-2-eth-1-yl)-8a-homoerthromycin A;
9-Deoxo-8a-aza-8a-((4-morpholinyl)-2-eth-1-yl)-8a-homoerthromycin A;
9-Deoxo-8a-aza-8a-((2-fluoroeth-1-yl)-2-aminoeth-1-yl)-8a-homoerthromycin A;
8a-(2-chloroallyl)-8a-aza-9-deoxo-8a-homoerythromycin A; 8a-(2-fluoroallyl)-8a-aza-9-deoxo-8a-homoerythromycin A;
9-Deoxo-8a-aza-8a-((2-cyano)eth-1-yl)-8a-homoerythromycin A;
9-Deoxo-8a-aza-8a-((3-amino)prop-1-yl)-8a-homoerythromycin A;
9-Deoxo-8a-aza-8a-(((N,N-dimethyl)-3-amino)prop-1-yl)-8a-homoerythromycin A;
9-Deoxo-8a-aza-8a-((2-cyanoethyl)-3-aminoprop-1-yl)-8a-homoerythromycin A;
9-Deoxo-8a-aza-8a-((3,4-dihydroxybenzyl)-3-aminoprop-1-yl)-8a-homoerythromycin A;
9-Deoxo-8a-aza-8a-(3-acetoxyprop-1-yl)-8a-homoerythromycin A;
9-Deoxo-8a-aza-8a-(3-hydroxyprop-1-yl)-8a-homoerythromycin A;
(3-methoxy-3-oxopropyl)-8a-homoerythromycin A;
8a-(3-octyloxy-3-oxopropyl)-9-deoxo-8a-aza-8a-homoerythromycin A;
8a-(3-(2-methoxyethoxy)-3-oxopropyl)-9-deoxo-8a-aza-8a-homoerthromycin A;
8a-(3-isopropoxy-3-oxopropyl)-9-deoxo-8a-aza-8a-homoerythromycin A;
8a-(3-benzyloxy-3-oxopropyl)-9-deoxo-8a-aza-8a-homoerthromycin A;
8a-(2-carboxyethyl)-9-deoxo-8a-aza-8a-homoerythromycin A;
9-Deoxo-8a-aza-8a-cyanomethyl-8a-homoerythromycin A;
9-Deoxo-8a-aza-8a-(2-aminoethyl)-8a-homoerythromycin A;
9-Deoxo-8a-aza-8a-(2-dimethylamino ethyl)-8a-homoerythromycin A;
9-Deoxo-8a-aza-8a-(N-L-leucyl-2-aminoethyl)-8a-homoerythromycin A;
9-Deoxo-8a-aza-8a-carboxymethyl-8a-homoerythromycin A;
9-Deoxo-8a-aza-8a-methoxycarbonylmethyl-8a-homoerythromycin A;
9-Deoxo-8a-aza-3'-N-demthyl-8a-homoerythromycin A.
9-Deoxo-8a-aza-3'-N-demethyl-3'-N-phenylsulfonyl-8a-homoerythromycin A;
9-Deoxo-8a-aza-8a-(2-fluoroeth-1-yl)-3'-N-demethyl-3'-N-phenylsulfonyl-8a-homoerythromycin A;
9-Deoxo-8a-aza-8a-(2-fluoroeth-1-yl)-3'-N-demethyl-8a-homoerythromycin A;
9-Deoxo-8a-aza-8a-(2-fluoroeth-1-yl)-8a-homerythromycin A;
9-Deoxo-8a-aza-8a-(3-fluoroprop-1-yl)-3'-N-demethyl-3'-N-phenylsulfonyl-8a-homoerythromycin A;
9-Deoxo-8a-aza-8a-(3-fluoroprop-1-yl)-3'-N-demethyl-8a-homoerythromycin A;
9-Deoxo-8a-aza-8a-(3-fluoroprop-1-yl)-8a-homoerythromycin A;
9-Deoxo-8a-aza-8a-((4,4,4-trifluoro)-but-1-yl)-3'-N-demethyl-3'-N-phenylsulfonyl-8a-homoerythromycin A;
9-Deoxo-8a-aza-8a-((4,4,4-trifluoro)but-1-yl)-3'-N-8a-homoerythromycin A;
9-Deoxo-8a-aza-8a-((4,4,4-trifluoro)but-1-yl)-8a-homoerythromycin A;
9-Deoxo-8a-aza-8a-(benzyl)-3'-N-demethyl-3'-N-phenylsulfonyl-8a-homoerythromycin A;
9-Deoxo-8a-aza-8a-(benzyl)-3'-N-demethyl-8a-homoerythromycin A;
9-Deoxo-8a-aza-8a-(benzyl)-8a-homoerythromycin A;
9-Deoxo-8a-aza-8a-(4-methoxybenzyl)-3'N-demethyl-3'-N-phenylsulfonyl-8a-homoerythromycin A;
9-Deoxo-8a-aza-8a-(4-methoxybenzyl)-3'-N-demethyl-8a-homoerythroycin A;
9-Deoxo-8a-aza-8a-(4-methoxybenzyl)-8a-homoerythromycin A;
9-Deoxo-8a-aza-8a-(2-(2-ethoxyethoxy)eth-1-yl)3'-N-demethyl-3'-N-phenylsulfonyl-8a-homoerythromycin A;
9-Deoxo-8a-aza-8a-(2-(2-ethoxyethoxy)eth-1-yl)-8a-homoerythromycin A;
9-Deoxo-8a-aza-8a-(2,2-difluoroeth-1-yl)-3'-N-demethyl-3'-N-phenylsulfonyl-8a-homoerythromycin A;
9-Deoxo-8a-aza-8a-(2,2-difluoroeth-1-yl)-3'-N-demethyl-8a-homoerythromycin A;
9-Deoxo-8a-aza-8a-(2,2-difluoroeth-1-yl)-8a-homoerythromycin A;
9-Deoxo-8a-aza-8a-hydroxy-8a-homoerythromycin A and 9-Deoxo-8a-aza-8a-(propl-yl)-8a-(propl-yl)-8a-homoerythromycin A;
9-Deoxo-8a-aza-8a-acetyl-8a-homoerythromycin A;
9-Deoxo-8a-aza-8a-glycyl-8a-homoerythromycin A;
9-Deoxo-8a-aza-8a-(Leu-Gly)-8a-homoerythromycin A;
9-Deoxo-8a-aza-8a-phenylsulfonyl-8a-homoerythromycin A;
2'-O-Acetyl-9-deoxo-8a-methyl-8a-aza-8a-homoerythromycin A;
(11-O,12-O-Oxomethylene)-9-deoxo-8a-methyl-8a-aza-8a-homoerythromycin A;
4"-O-phenylacetyl-8a-aza-8a-methyl-9-deoxo-8a-homoerythromycin A of 8a-aza;
4"-O-(4-methoxyphenyl)-acetyl-8a-aza-8a-methyl-9-deoxo-8a-homoerythromycin A;
2'-O-Acetyl-9-deoxo-8a-aza-8a-methyl-8a-homoerythromycin A;
4"-epi-9-deoxo-8a-aza-8a-methyl-8a-homoerythromycin A;
4"-Deoxy-4"-amino-9-deoxo-8a-aza-8a-methyl-8a-homoerythromycin A;
4"-Deoxy-4"-(S)-amino-9-deoxo-8a-aza-8a-methyl-8a-homoerythromycin A;
4"-Deoxy-4"-(R)-amino-9-deoxo-8a-aza-8a-methyl-8a-homoerythromycin A;
4"-Deoxo-4"-(S)-acetylamino-9-deoxo-8a-aza-8a-methyl-8a-homoerythromycin A;
4"-Deoxo-4"-(R)-acetylamino-9-deoxo-8a-aza-8a-methyl-8a-homoerythromycin A;
4"-(4-methoxyphenylacetyl)amino-4"-deoxo-8a-aza-8a-methyl-8a-homoerythromycin A;
4"-Deoxy-4"-(L-alanyl)amino-9-deoxo-8a-aza-8a-methyl-8a-methyl-8a-homoerythromycin A;
4"-Deoxy-4"-(L-valyl)amino-9-deoxo-8a-aza-8a-methyl-8a-homoerythromycin A;
4"-Deoxy-4"-(L-leucyl)amino-9-deoxo-8a-aza-8a-methyl-8a-homoerythromycin A;
4"-Deoxy-4"-(L-phenylalanyl)amino-9-deoxo-8a-aza-8a-methyl-8a-homoerythromycin A;
4"-Deoxy-4"-(O-tert-butyl-L-tyrosyl)amino-9-deoxo-8a-aza-8a-methyl-8a-homoerythromycin A;
4"-Deoxy-4"-(L-propyl)amino-9-deoxo-8a-aza-8a-methyl-8a-homoerythromycin A;
4"-Deoxy-4"-(L-aspartyl-b-benzyl ester)-amino-9-deoxo-8a-aza-8a-methyl-8a-homoerythromycin A;
4"-Deoxy-4"-(L-aspartyl)amino-9a-deoxo-8a-aza-8a-methyl-8a-homoerythromycin A;
4"-Deoxy-4"-(L-pyroglutamyl)amino-9-deoxo-8a-aza-8a-methyl-8a-homoerythromycin A;
4"-Deoxy-4"-(L-glutamyl)amino-9-deoxo-8a-aza-8a-methyl-8a-homoerythromycin A;
2"-O-Acetyl-9-deoxo-8a-aza-8a-allyl-8a-homoerythromycin A;
4"-Deoxy-4-amino-9-deoxo-8a-aza-8a-allyl-8a-homoerythromycin A;
4"-Deoxy-4"-amino-9-deoxo-8a-aza-8a-propyl-8a-homoerythromycin A;
2"-O-Acetyl-9-deoxo-8a-aza-8a-methoxycarbonethyl-8a-homoerythromycin A;
4"-Deoxy-4"-amino-9-deoxo-8a-aza-8a-(3-methoxy-3-oxopropyl)-8a-homoerythromycin A;
2'-O-Acetyl-8a-aza-8a-homoerythromycin; and
4"-Deoxy-4"-amino-8a-aza-8a-homoerythromycin A.

8a-Azalides may be administered to a host in need of treatment for, or prevention of bacterial respiratory or enteric diseasease in a manner similar to that used for other antibacterial agents; for example, 8a-azalides may be administered parenterally, orally, topically, or rectally. The dosage to be administered will vary according to the particular compound used, the infectious organism involved, the particular host, the severity of the disease, physical condition of the host, and the selected route of administration; the appropriate dosage can be readily determined by a person skilled in the art. For the treatment of bacterial diseases, the oral dosage may range from 1 mg/kg to 1000 mg/kg; and the parenteral dosage may range from 0.01 mg/kg to 500 mg/kg. For prophylactic use in animals, the oral dosage may range from 1 mg/kg to 1000 mg/kg; and the parenteral dosage may range from 0.01 mg/kg to 500 mg/kg. The 8a-azalides of the present invention are preferably administered parenterally at a dosage range of about 0.1 to about 10 mg/kg.

The 8a-azalides are preferably used in a pharmaceutical composition comprising the active ingredient and an inert pharmaceutically acceptable carrier. The pharmaceutical compositions of the present invention comprise an 8a-azalide as an active ingredient, and may also contain a pharmaceutically acceptable carrier and optionally other therapeutic ingredients. The formulations include compositions suitable for oral, rectal, topical, and parenteral (including subcutaneous, intramuscular, and intravenous) administrations, although the most suitable route in any given case will depend on the particular host, and nature and severity of the conditions for which the active ingredient is being administered. The pharmaceutical compositions may be conveniently presented in unit dosage form and prepared by any of the methods well-known in the art of pharmacy.

In practical use, the 8a-azalide can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous, intramuscular, and subcutaneous); generally parenteral administration is preferred.

In preparing the compositions for oral dosage form, any of the usual pharmaceutical media may be employed. For example, in the case of oral liquid preparations such as suspensions, elixirs and solutions, water, glycols, oils, alcohols, flavoring agents, preservatives, coloring agents and the like may be used; or in the case of oral solid preparations such as powders, capsules and tablets, carriers such as starches, sugars, microcrystalline cellulose, diluents, granulating agents, lubricants, binders, disintegrating agents, and the like may be included. Because of their ease of administration, tablets and capsules represent the most advantageous oral dosage unit form in which case solid pharmaceutical carriers are obviously employed. If desired, tablets may be coated by standard aqueous or nonaqueous techniques. In addition to the common dosage forms set out above, 8a-azalides may also be administered by controlled release means and/or delivery devices.

Pharmaceutical compositions of the present invention suitable for oral administration may be presented as discrete units such as capsules, cachets or tablets each containing a predetermined amount of the active ingredient, as a powder or granules or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or molding, optionally with one or more accessory ingredients.
Compressed tablets may be prepared by compressing, in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moistened with an inert liquid diluent. Desirably, each tablet contains from about 1 mg to about 500 mg of the active ingredient and each cachet or capsule contains from about 1 to about 500 mg of the active ingredient.

Pharmaceutical compositions of the present invention suitable for parenteral administration may be prepared as solutions or suspensions of these active compounds in water suitably mixed with a surfactant such as hydroxypropylcellulose. Dispersions can also be prepared in glycerol, liquid polyethylene glycols, and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g. glycerol, propylene glycol and liquid polyethylene glycol), suitable mixtures thereof, and vegetable oils.

Suitable topical formulations include transdermal devices, aerosols, creams, ointments, lotions, dusting powders, and the like. These formulations may be prepared via conventional methods containing the active ingredient. To illustrate, a cream or ointment is prepared by mixing sufficient quantities of hydrophilic material and water, containing from about 5-10% by weight of the compound, in sufficient quantities to produce a cream or ointment having the desired consistency.

Pharmaceutical compositions suitable for rectal administration wherein the carrier is a solid are most preferably presented as unit dose suppositories. Suitable carriers include cocoa butter and other materials commonly used in the art, and the suppositories may be conveniently formed by admixture of the combination with the softened or melted carrier(s) followed by chilling and shaping moulds.

It should be understood that in addition to the aforementioned carrier ingredients the pharmaceutical formulations described above may include, as appropriate, one or more additional carrier ingredients such as diluents, buffers, flavoring agents, binders, surface-active agents, thickeners, lubricants, preservatives (including anti-oxidants) and the like, and substances included for the purpose of rendering the formulation isotonic with the blood of the intended recipient.

Compositions containing an 8a-azalide may also be prepared in powder or liquid concentrate form. In accordance with standard veterinary formulation practice, conventional water soluble excipients, such as lactose or sucrose, may be incorporated in the powders to improve their physical properties. Thus particularly suitable powders of this invention comprise 50 to 100% w/w, and preferably 60 to 80% w/w of the combination and 0 to 50% w/w and preferably 20 to 40% w/w of conventional veterinary excipients. These powders may either be added to animal feedstuffs, for example by way of an intermediate premix, or diluted in animal drinking water.

Liquid concentrates of this invention suitably contain a water-soluble compound combination and may optionally include a veterinarily acceptable water miscible solvent, for example polyethylene glycol, propylene glycol, glycerol, glycerol formal or such a solvent mixed with up to 30% v/v of ethanol. The liquid concentrates may be administered to the drinking water of animals.

The pharmaceutical composition containing 8a-azalide may optionally contain a second active ingredient, a biological component such as an antigen, or a dietary supplement such as minerals or vitamins. Active ingredients may include immunomodulators such as interferon, interleukins and other chemokines, non-steroidal antiinflammatories such as propionic acid derivatives (e.g. ibuprofen, ketoprofen, naproxen, benoxprofen, carprofen), acetic acid derivatives (e.g. acemetacin, alclofenac, clidanac, diclofenac, fenclofenac, fenclozic acid, fentiazac, furofenac, ibufenac, isoxepac, oxpinac, sulindac, tiopinac, tolmetin, zidometacin, and zomepirac), fenamic acid derivatives (e.g. flufenamic acid, meclofenamic acid, mefenamic acid, niflumic acid and tolfenamic acid), biphenylcarboxylic acids (e.g. diflufenisal, flufenisal), and cyclooxygenase-2 (COX-2) inhibitors, and antiparasitic agents such as avermectin, ivermectins, milbemycins, levamisole, benzimidazoles, pyrantel/morantel. Biologicals may be vaccines commonly used in the livestock industry against infectious bovine rhinotracheitis, bovine virus diarrhea, respiratory syncythial virus, parainfluenza, transmissable gastroenteritis, porcine reproductive and respiratory syndrome, rotavirus, and coronavirus. Dietary supplements may be vitamins, iron, selenium, and the like.

The following examples are provided to more fully illustrated the present invention, and are not be construed as limiting the scope of the claims in any manner.

### IN VITRO ACTIVITY OF 8a-AZALIDES

The antibacterial activity of representative 8a-azalides against a panel of veterinary pathogens was determined by the minimum inhibitory concentration (MIC) method well known in the art. This is done by preparing a series of culture tubes, each containing a medium with a different concnetration of the antimicrobial agent, and inoculating all the tubes with the same organism. The lowest concentration of agent that completely prevents the appearance of turbidity is noted, and this concentration is called the MIC.

The range of antibacterial activity of 4"-deoxy-4"-amino-9-deoxo-8a-aza-8a-methyl-8a-homoerythromycin A, 4"-deoxy-4"(R)-amino-9-deoxo-8a-aza-8a-methyl-8a-homoerythromycin A, 4"-deoxy-4"(S)-amino-9-deoxo-8a-aza-8a-methyl-8a-homoerythromycin A, and 4"-deoxy-4"-amino-9-deoxo-8a-aza-8a-allyl-8a-homoerythromycin A against key veterinary organisms is summarized below:

| Organism | MIC range (µg/ml) |
|---|---|
| *P. haemolytica* | 0.125 - 0.5 |
| *P. multocida* | 0.125 - 0.5 |
| *H. somnus* | 0.125 - 0.250 |
| *A. pleuropneumoniae* | 0.062 - 0.125 |
| *E. coli* | 0.5 - 2 |
| *Salmonella* spp. | 0.5 - 4 |

## Claims

1. The use of an 8a-azalide for the manufacture of a medicament for the treatment or prevention of a bacterial bovine or swine respiratory infection caused by a *Pasteurella* spp., an *Actircobacillus* spp., *Haemophilus somnus* or *Mycoplasma* spp.

2. The use of an 8a-azalide for the manufacture of a medicament for the treatment or prevention of a bacterial bovine or swine enteric infection.

3. The use as claimed in claim 2 wherein said enteric infection is caused by *E*. *coli*, *Treponema hyodysenteriae* or a *Salmonella* spp.

4. The use as claimed in any one of the preceding claims wherein the 8a-azalide has the formula I: 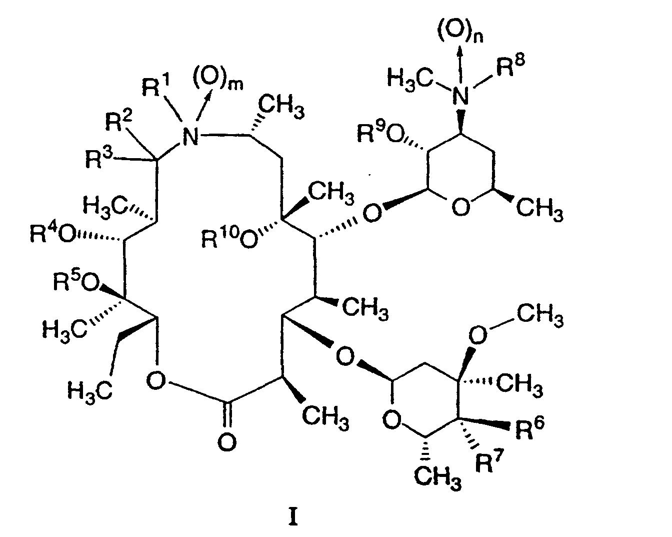 or a pharmaceutically acceptable salt thereof, or a pharmaceutically acceptable metal complex thereof, and said metal complex is taken from the group consisting of copper, zinc, cobalt, nickel and cadmium; where
R¹ is hydrogen;
hydroxy;
C₁₋₄ alkoxy;
formyl;
C₁₋₁₀ alkylcarbonyl, C₁₋₁₀ alkoxycarbonyl, aryloxycarbonyl, C₁₋₁₀ aralkoxycarbonyl, C₁₋₁₀ alkylsulfonyl, or arylsulfonyl wherein said C₁₋₁₀ alkyl group is substituted by 1-3 halo (F,Cl,Br), hydroxy, amino, C₁₋₅ acylamino or C₁₋₄ alkyl groups; or
unsubstituted or substituted C₁₋₁₀ alkyl, C₂₋₁₀ alkenyl or C₂₋₁₀ alkynyl wherein said alkyl chain, if more than 2 carbons in length, can have inserted therein 1 to 2 of oxa, thia or aza of the formula -NR-where R is hydrogen or C₁₋₃ alkyl, and wherein said substituents are independently 1-3 of
(a) aryl or heteroaryl optionally substituted by 1-3 halo (F, Cl, Br, I), C₁₋₄ alkyl, C₁₋₃ alkoxy, amino, C₁₋₄ alkylamino, di(C₁₋₄ alkyl)amino or hydroxy,
(b) heterocyclyl unsubstituted or substituted by hydroxy, amino, C₁₋₄ alkylamino, di(C₁₋₄ alkyl) amino, C₁₋₄ alkylcarbonyloxy or C₁₋₄ alkylcarbonylamino,
(c) halo (F,Cl,Br or I),
(d) hydroxy non-acylated or acylated by a group R^{a}C(=O) or R^{b} S(O)2 wherein R^{a} is hydrogen, C₁₋₆ alkyl, aryl, heteroaryl, aralkyl, or heteroaralkyl and R^{b} is C₁₋₆ alkyl or aryl,
(e) C₁₋₁₀ alkoxy,
(f) aryloxy or heteroaryloxy unsubstituted or substituted by 1-3 halo, hydroxy, amino or C₁₋₄ alkyl groups,
(g) amino or C₁₋₁₀ alkylamino non-acylated or acylated by a group R^{a}C(=O), R^{a}OC(=O), or R^{b}SO₂, wherein R^{a} and R^{b} are as defined above;
(h) di(C₁₋₁₀ alkyl)amino,
- (i) arylamino, heteroarylamino, aralkylamino or heteroarylalkylamino wherein said aryl or heteroaryl group is unsubstituted or substituted by 1-3 halo, hydroxy, amino or C₁-C₄ alkyl groups,
(j) mercapto,
(k) C₁₋₁₀ alkylthio, alkylsulfinyl or alkylsulfonyl, arylthio, arylsulfinyl or arylsulfonyl wherein said aryl group is unsubstituted or substituted by 1-3 halo, hydroxy, amino or C₁₋₄ alkyl groups,
(l) formyl,
(m) C₁₋₁₀ alkylcarbonyl,
(n) arylcarbonyl, heteroarylcarbonyl, aralkylcarbonyl or heteroarylalkylcarbonyl wherein said aryl or heteroaryl group is unsubstituted or substituted by 1-3 halo, hydroxy, amino or C-₄ alkyl groups,
(o) carboxy,
(p) C₁₋₁₀ alkoxycarbonyl,
(q) aryloxycarbonyl, heteroaryloxycarbonyl, aralkoxycarbonyl or heteroarylalkoxycarbonyl wherein said aryl or heteroaryl group is unsubstituted or substituted by 1-3 halo, hydroxy, amino or C₁₋₄ alkyl groups,
(r) carbamoyl or sulfamoyl wherein the N-atom is unsubstituted or substituted by 1-2 C₁₋₆ alkyl groups or by a C₄₋₆ alkylene chain,
(s) cyano,
(t) isonitrilo
(u) nitro,
(v) azido,
(w) iminomethyl unsubstituted or substituted on nitrogen or carbon with C₁₋₁₀ alkyl,
(x) oxo or
(y) thiono;
R² and R³ are independently
hydrogen,
C₁₋₁₀ alkyl,
aryl; or
R² and R³ together are oxo or thiono;
R⁴ and R⁵ are independently hydrogen or alkylcarbonyl; or
R⁴ and R⁵ are together carbonyl; or
R⁴ and R¹ together are C₁-C₃ alkylene unsubstituted or substituted by an oxo group;
R⁶ and R⁷ are both hydrogen, or
one of R⁶ and R⁷ is hydrogen and the other is hydroxy,
an acyloxy derivative taken from the group consisting of formyloxy, C₁₋₁₀ alkylcarbonyloxy, arylcarbonyloxy and aralkylcarbonyloxy, or
-NHR¹² wherein R¹² is hydrogen, arylsulfonyl or heteroarylsulfonyl unsubstituted or substituted by 1-3 halo or C₁₋₃ alkyl groups, alkylsulfonyl or - C(=O)-X-A-R¹³ where X is a connecting bond, O or NH, A is a connecting bond or C₁-C₃ alkylene, R¹³ is hydrogen, C₁₋ C₁₀ alkyl, aryl, aralkyl, heteroaryl, heterocyclyl, or C₃-C₇ cycloalkyl, any of which R¹³ groups other than hydrogen can be substituted by one or more of halogen, hydroxyl, C₁-C₃ alkoxy, cyano, isonitrilo, nitro, amino, mono- or di- (C₁-C₃) alkylamino, mercapto, C₁-C₃ alkylthio, C₁-C₃ alkylsulfinyl, C₁-C₃ alkylsulfonyl, arylthio, arylsulfinyl, sulfamoyl, arylsulfonyl, carboxy, carbamoyl, C₁-C₃ alkylcarbonyl, or C₁-C₃ alkoxycarbonyl; or
R⁶ and R⁷ are together oxo, hydroxyimino, alkoxyimino, aralkoxyimino or aminoimino;
R⁸ is methyl,
aralkoxycarbonyl, or
arylsulfonyl;
R⁹ is hydrogen,
formyl,
C₁₋₁₀ alkylcarbonyl,
C₁₋₁₀ alkoxycarbonyl, or
arylalkoxycarbonyl;
R¹⁰ is hydrogen; or
R¹⁰ and R¹ together are C₁-C₃ alkylene unsubstituted or substituted by an oxo group;
m and n are independently zero or one.

5. The use as claimed in Claim 4 wherein the 8a-azalide has the formula I wherein
n and m are zero;
R¹ is hydrogen, C₁₋₁₀ alkyl, C₂₋₁₀ alkenyl, C₂₋₁₀ alkynyl or arylsulfonyl, wherein said alkyl and alkenyl are optionally substituted with halo, hydroxy, cyano, C₁₋₁₀ alkoxycarbonyl, amino, C₁₋₁₀ alkylamino, di(C₁₋₁₀ alkylamino), aryl or aralkoxycarbonyl;
R², R³, R⁴, R⁵, R⁹ and R¹⁰ are each hydrogen;
one of R6 and R7 is hydrogen and the other is selected from hydroxyl, C₁₋ ₁₀ alkyl carbonyloxy, aralkylcarbonyloxy, amino, amino substituted by C₁₋₁₀ alkylcarbonyl, arylcarbonyl, aryl C₁₋₁₀ alkylcarbonyl, C₁₋₁₀ alkoxycarbonyl, aryl C₁₋₁₀ alkoxycarbonyl, heteroarylcarbonyl, heteroarylalkylcarbonyl or arylsulfonyl;
R8 is methyl.

6. The use as claimed in Claim 4 wherein the 8a-azalide has the formula I wherein
n and m are zero;
R¹ is methyl, ethyl, propyl, allyl, propargyl, 2-cyanoethyl, 2-hydroxyethyl, 3-hydroxypropyl, 2-methoxycarbonylethyl, 2-benzyloxycarbonylethyl, cyanomethyl, 2-aminoethyl, 2-(dimethylamino)ethyl, 2-fluoroethyl, 2-fluoroallyl, benzyl or oxiranylmethyl;
R², R³, R⁴, R⁵, R⁹ and R¹⁰ are hydrogen;
one of R6 or R7 is hydrogen and the other is hydroxy or amino;
R8 is methyl.

7. The use as claimed in Claim 6 wherein R¹ is methyl, ethyl, propyl, allyl, 2-methoxycarbonylethyl or 2-(dimethylamino)ethyl.

## Patentansprüche

1. Die Verwendung eines 8a-Azalids zur Herstellung eines Medikaments zur Behandlung oder Prävention einer bakteriellen Infektion der Atemwege beim Rind oder Schwein, hervorgerufen durch *Pasteurella* spp., *Actinobacillus* spp., *Haemophilus somnus* oder *Mycoplasma* spp.

2. Die Verwendung eines 8a-Azalids zur Herstellung eines Medikaments zur Behandlung oder Prävention einer bakteriellen Darminfektion beim Rind oder Schwein.

3. Die wie in Anspruch 2 beanspruchte Verwendung, wobei die Darminfektion durch *E. coli, Treponema hyodysenteriae* oder eine *Salmonella* spp. hervorgerufen wird.

4. Die wie in irgendeinem der vorhergehenden Ansprüche beanspruchte Verwendung, wobei das 8a-Azalid die Formel I hat: 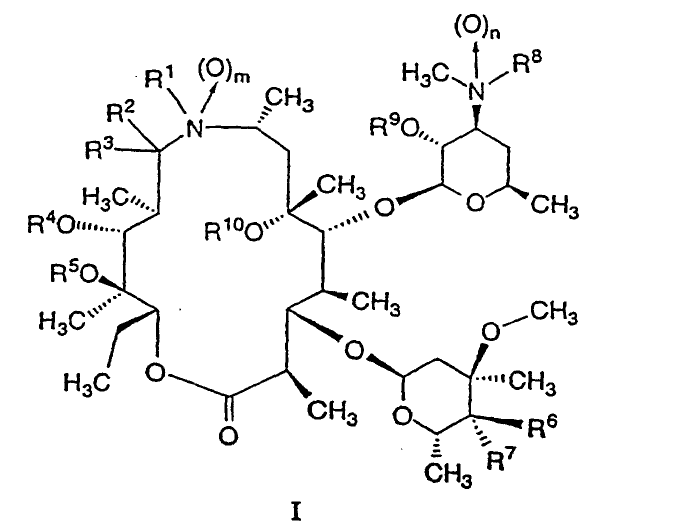 oder ein pharmazeutisch annehmbares Salz davon oder ein pharmazeutisch annehmbarer Metallkomplex davon ist und der Metallkomplex ausgewählt ist aus der Gruppe, bestehend aus Kupfer, Zink, Cobalt, Nickel und Cadmium, wobei
R¹ Wasserstoff,
Hydroxy,
C₁₋₄-Alkoxy,
Formyl,
C₁₋₁₀-Alkylcarbonyl, C₁₋₁₀-Alkoxycarbonyl, Aryloxycarbonyl, C₁₋₁₀-Aralkoxycarbonyl, C₁₋₁₀-Alkylsulfonyl oder Arylsulfonyl, wobei die C₁₋₁₀-Alkylgruppe durch 1-3 Halogen- (F, Cl, Br), Hydroxy-, Amino-, C₁₋₅-Acylamino- oder C₁₋₄-Alkylgruppen substituiert ist, oder
unsubstituiertes oder substituiertes C₁₋₁₀-Alkyl, C₂₋₁₀-Alkenyl oder C₂₋₁₀-Alkinyl ist, wobei in die Alkylkette, wenn ihre Länge mehr als 2 Kohlenstoffe beträgt, 1 bis 2 Oxa, Thia oder Aza der Formel -NR-, wobei R Wasserstoff oder C₁₋₃-Alkyl ist, eingefügt sein können und wobei die Substituenten unabhängig 1-3 von
(a) Aryl oder Heteroaryl, gegebenenfalls substituiert durch 1-3 Halogen (F, Cl, Br, I), C₁₋₄-Alkyl, C₁₋₃-Alkoxy, Amino, C₁₋₄-Alkylamino, Di(C₁₋₄-alkyl)amino oder Hydroxy,
(b) Heterocyclyl, unsubstituiert oder substituiert durch Hydroxy, Amino, C₁₋₄-Alkylamino, Di(C₁₋₄-alkyl)amino, C₁₋₄-Alkylcarbonyloxy oder C₁₋₄-Alkylcarbonylamino,
(c) Halogen (F, Cl, Br oder I),
(d) Hydroxy, nichtacyliert oder acyliert durch eine Gruppe R^{a}C(=O) oder R^{b}S(O)₂, wobei R^{a} Wasserstoff, C₁₋₆-Alkyl, Aryl, Heteroaryl, Aralkyl oder Heteroaralkyl ist und R^{b} C₁₋₆-Alkyl oder Aryl ist,
(e) C₁₋₁₀-Alkoxy,
(f) Aryloxy oder Heteroaryloxy, unsubstituiert oder substituiert durch 1-3 Halogen-, Hydroxy-, Amino- oder C₁₋₄-Alkylgruppen,
(g) Amino oder C₁₋₁₀-Alkylamino, nichtacyliert oder acyliert durch eine Gruppe R^{a}C(=O), R^{a}OC(=O) oder R^{b}SO₂, wobei R^{a} und R^{b} wie oben definiert sind,
(h) Di(C₁₋₁₀-alkyl)amino,
(i) Arylamino, Heteroarylamino, Aralkylamino oder Heteroarylalkylamino, wobei die Aryl- oder Heteroarylgruppe unsubstituiert oder substituiert ist durch 1-3 Halogen-, Hydroxy-, Amino- oder C₁-C₄-Alkylgruppen,
(j) Mercapto,
(k) C₁₋₁₀-Alkylthio, Alkylsulfinyl oder Alkylsulfonyl, Arylthio, Arylsulfinyl oder Arylsulfonyl, wobei die genannte Arylgruppe unsubstituiert oder substituiert ist durch 1-3 Halogen-, Hydroxy-, Amino- oder C₁₋₄-Alkylgruppen,
(l) Formyl,
(m) C₁₋₁₀-Alkylcarbonyl,
(n) Arylcarbonyl, Heteroarylcarbonyl, Aralkylcarbonyl oder Heteroarylalkylcarbonyl, wobei die Aryl- oder Heteroarylgruppe unsubstituiert oder substituiert ist durch 1-3 Halogen-, Hydroxy-, Amino- oder C₋₄-Alkylgruppen,
(o) Carboxy,
(p) C₁₋₁₀-Alkoxycarbonyl,
(q) Aryloxycarbonyl, Heteroaryloxycarbonyl, Aralkoxycarbonyl oder Heteroarylalkoxycarbonyl, wobei die Aryl- oder Heteroarylgruppe unsubstituiert oder substituiert ist durch 1-3 Halogen-, Hydroxy-, Amino- oder C₁₋₄-Alkylgruppen,
(r) Carbamoyl oder Sulfamoyl, wobei das N-Atom unsubstituiert oder substituiert ist durch 1-2 C₁₋₆-Alkylgruppen oder durch eine C₄₋₆-Alkylenkette,
(s) Cyano,
(t) Isonitrilo,
(u) Nitro,
(v) Azido,
(w) Iminomethyl, unsubstituiert oder am Stickstoff oder Kohlenstoff mit C₁₋₁₀-Alkyl substituiert,
(x) Oxo oder
(y) Thiono sind,
R² und R³ unabhängig
Wasserstoff,
C₁₋₁₀-Alkyl,
Aryl sind oder
R² und R³ zusammen Oxo oder Thiono sind,
R⁴ und R⁵ unabhängig Wasserstoff oder Alkylcarbonyl sind oder
R⁴ und R⁵ zusammen Carbonyl sind oder
R⁴ und R¹ zusammen C₁-C₃-Alkylen sind, unsubstituiert oder substituiert durch eine Oxo-Gruppe,
R⁶ und R⁷ beide Wasserstoff sind oder
einer der Reste R⁶ und R⁷ Wasserstoff ist und der andere ist Hydroxy,
ein Acyloxyderivat, ausgewählt aus der Gruppe, bestehend aus Formyloxy, C₁₋₁₀-Alkylcarbonyloxy, Arylcarbonyloxy und Aralkylcarbonyloxy, oder
-NHR¹², wobei R¹² ist Wasserstoff, Arylsulfonyl oder Heteroarylsulfonyl, unsubstituiert oder substituiert durch 1-3 Halogenoder C₁₋₃-Alkylgruppen, Alkylsulfonyl oder -C(=O)-X-A-R¹³, wobei X eine verbindende Bindung, O oder NH ist, A eine verbindende Bindung oder C₁-C₃-Alkylen ist, R¹³ Wasserstoff, C₁₋₁₀-Alkyl, Aryl, Aralkyl, Heteroaryl, Heterocyclyl oder C₃-C₇-Cycloalkyl ist, wobei jede dieser R¹³-Gruppen, wenn sie anders als Wasserstoff ist, durch ein oder mehrere Halogen, Hydroxyl, C₁-C₃-Alkoxy, Cyano, Isonitrilo, Nitro, Amino, Mono- oder Di(C₁-C₃)alkylamino, Mercapto, C₁-C₃-Alkylthio, C₁-C₃-Alkylsulfinyl, C₁-C₃-Alkylsulfonyl, Arylthio, Arylsulfinyl, Sulfamoyl, Arylsulfonyl, Carboxy, Carbamoyl, C₁-C₃-Alkylcarbonyl oder C₁-C₃-Alkoxycarbonyl substituiert sein kann, oder
R⁶ und R⁷ zusammen Oxo, Hydroxyimino, Alkoxyimino, Aralkoxyimino oder Aminoimino sind,
R⁸ Methyl,
Aralkoxycarbonyl oder
Arylsulfonyl ist,
R⁹ Wasserstoff,
Formyl,
C₁₋₁₀-Alkylcarbonyl,
C₁₋₁₀-Alkoxycarbonyl oder
Arylalkoxycarbonyl ist,
R¹⁰ Wasserstoff ist oder
R¹⁰ und R¹ zusammen C₁-C₃-Alkylen sind, unsubstituiert oder substituiert durch eine Oxo-Gruppe,
m und n unabhängig null oder eins sind.

5. Die wie in Anspruch 4 beanspruchte Verwendung, wobei das 8a-Azalid die Formel I hat, wobei
n und m null sind,
R¹ Wasserstoff, C₁₋₁₀-Alkyl, C₂₋₁₀-Alkenyl, C₂₋₁₀-Alkinyl oder Arylsulfonyl ist, wobei das Alkyl und Alkenyl gegebenenfalls substituiert sind mit Halogen, Hydroxy, Cyano, C₁₋₁₀-Alkoxycarbonyl, Amino, C₁₋₁₀-Alkylamino, Di(C₁₋₁₀-alkylamino), Aryl oder Aralkoxycarbonyl,
R², R³, R⁴, R⁵, R⁹ und R¹⁰ jeweils Wasserstoff sind,
einer der Reste R⁶ und R⁷ Wasserstoff ist und der andere ausgewählt ist aus Hydroxyl, C₁₋₁₀-Alkylcarbonyloxy, Aralkylcarbonyloxy, Amino, durch C₁₋₁₀-Alkylcarbonyl substituiertes Amino, Arylcarbonyl, Aryl-C₁₋₁₀-alkylcarbonyl, C₁₋₁₀-Alkoxycarbonyl, Aryl-C₁₋₁₀alkoxycarbonyl, Heteroarylcarbonyl, Heteroarylalkylcarbonyl oder Arylsulfonyl,
R⁸ Methyl ist.

6. Die wie in Anspruch 4 beanspruchte Verwendung, wobei das 8a-Azalid die Formel I besitzt, wobei
n und m null sind,
R¹ Methyl, Ethyl, Propyl, Allyl, Propargyl, 2-Cyanoethyl, 2-Hydroxyethyl, 3-Hydroxypropyl, 2-Methoxycarbonylethyl, 2-Benzyloxycarbonylethyl, Cyanomethyl, 2-Aminoethyl, 2-(Dimethylamino)-ethyl, 2-Fluorethyl, 2-Fluorallyl, Benzyl oder Oxiranylmethyl ist,
R², R³, R⁴, R⁵, R⁹ und R¹⁰ Wasserstoff sind,
einer der Reste R⁶ oder R⁷ Wasserstoff ist und der andere Hydroxy oder Amino ist,
R⁸ Methyl ist.

7. Die wie in Anspruch 6 beanspruchte Verwendung, wobei R¹ Methyl, Ethyl, Propyl, Allyl, 2-Methoxycarbonylethyl oder 2-(Dimethylamino)ethyl ist.

## Revendications

1. Utilisation d'un 8a-azalide pour la fabrication d'un médicament, pour le traitement ou la prévention d'une infection respiratoire bactérienne bovine ou de porc, provoquée par *Pasteurella* spp., *Actinobacillus* spp., *Haemophilus somnus* ou *Mycoplasma* spp.

2. Utilisation d'un 8a-azalide pour la fabrication d'un médicament pour le traitement ou la prévention d'une infection digestive bactérienne bovine ou de porc.

3. Utilisation selon la revendication 2, dans laquelle ladite infection digestive est provoquée par *E. coli, Treponema hyodysenteriae* ou *Salmonella* spp.

4. Utilisation selon l'une quelconque des revendications précédentes, dans laquelle le 8a-azalide a la formule I : 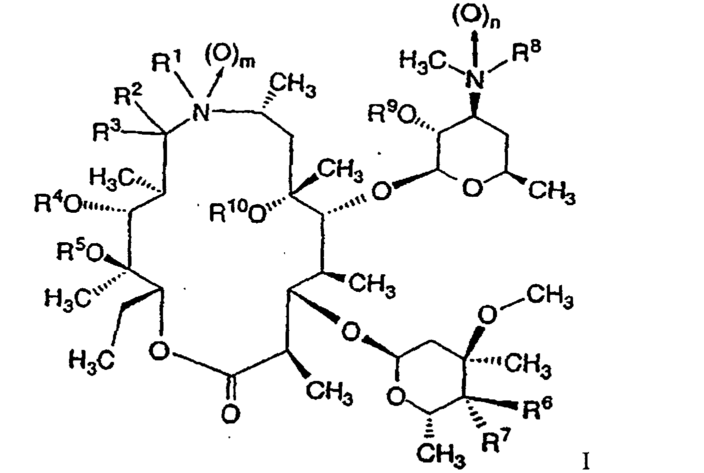 ou un sel pharmaceutiquement acceptable de celui-ci, ou un complexe métallique pharmaceutiquement acceptable de celui-ci, et ledit complexe métallique est choisi dans le groupe constitué par le cuivre, le zinc, le cobalt, le nickel et le cadmium; dans laquelle
R¹ est un hydrogène ;
un hydroxy ;
un alcoxy C₁₋₄ ;
un formyle;
un carbonylalkyle C₁₋₁₀, un alcoxycarbonyle C₁₋₁₀, un aryloxycarbonyle, un aralcoxycarbonyle C₁₋₁₀, un sulfonylalkyle C₁₋₁₀ ou un sulfonylaryle, dans lesquels ledit groupement alkyle C₁₋₁₀ est substitué par 1 à 3 groupements halogène (F, Cl ,Br), hydroxy, amino, acylamino C₁₋₅ ou alkyle C₁₋₄ ; ou
qui n'est pas substitué ou qui est substitué par un alkyle C₁₋₁₀, un alcényle C₂₋₁₀ ou un alcynyle C₂₋₁₀, dans lesquels ladite chaîne alkyle, si elle a plus de 2 atomes de carbone de longueur, peut avoir insérés dedans 1 à 2 oxa, thia ou aza de formule-NR-, dans lequel R est un hydrogène ou un alkyle C₁₋ ₃, et dans lesquels lesdits substituants sont indépendamment 1 à 3 de
(a) un aryle ou un hétéroaryle facultativement substitué par 1 à 3 halogène (F, Cl, Br, I), alkyle C₁₋₄, alcoxy C₁₋₃, amino, aminoalkyle C₁₋₄, aminodi (alkyle C₁₋₄) ou hydroxy,
(b) un groupement hétérocyclique qui n'est pas substitué ou qui est substitué par un hydroxy, un amino, un aminoalkyle C₁₋₄, un aminodi (alkyle C₁₋₄), un carbonyloxyalkyle C₁₋₄ ou un carbonylaminoalkyle C₁₋₄,
(c) un halogène (F, Cl, Br ou I),
(d) un hydroxy non acylé ou acylé par un groupement R^{a}C(=O) ou R^{b}S(O)₂ dans lesquels R^{a} est un hydrogène, un alkyle C₁₋₆, un aryle, un hétéroaryle, un aralkyle, ou un hétéroaralkyle et R^{b} est un alkyle C₁₋₆ ou un aryle,
(e) un alcoxy C₁₋₁₀,
(f) un aryloxy ou un hétéroaryloxy qui n'est pas substitué ou qui est substitué par 1 à 3 groupements halogène, hydroxy, amino ou alkyle C₁₋₄,
(g) un amino ou un aminoalkyle C₁₋₁₀ non acylé ou acylé par un groupement R^{a}C(=O), R^{a}OC(=O), ou R^{b}SO₂, dans lequel R^{a} et R^{b} sont tels que définis précédemment,
(h) un aminodi (alkyle C₁₋₁₀),
(i) un aminoaryle, un aminohétéroaryle, un aminoaralkyle ou un aminohétéroarylalkyle dans lesquels ledit groupement aryle ou hétéroaryle n'est pas substitué ou est substitué par 1 à 3 groupements halogène, hydroxy, amino ou alkyle C₁₋₄,
(j) un mercapto,
(k) un thioalkyle C₁₋₁₀, un sulfinylalkyle ou un sulfonylalkyle, un thioaryle, un sulfinylaryle ou un sulfonylaryle, dans lequel ledit groupement aryle n'est pas substitué ou est substitué par 1 à 3 groupements halogène, hydroxy, amino ou alkyle C₁₋₄,
(l) un formyle,
(m) un carbonylalkyle C₁₋₁₀,
(n) un carbonylaryle, un hétérocarbonylaryle, un carbonylaralkyle ou un carbonylhétéroarylalkyle dans lequel ledit groupement aryle ou hétéroaryle n'est pas substitué ou est substitué par 1 à 3 groupements halogène, hydroxy, amino ou alkyle C ₋₄,
(o) un carboxy,
(p) un alcoxycarbonyle C₁₋₁₀,
(q) un aryloxycarbonyle, un hétéroaryloxycarbonyle, un aralcoxycarbonyle ou un hétéroarylalcoxycarbonyle dans lesquels ledit groupement aryle ou hétéroaryle n'est pas substitué ou est substitué par 1 à 3 groupements halogène, hydroxy, amino ou alkyle C₁₋₄,
(r) un carbamoyle ou un sulfamoyle dans lequel l'atome N n'est pas substitué ou est substitué par 1 à 2 groupements alkyle C₁₋₆ ou par une chaîne alkylène C₄₋₆,
(s) un cyano,
(t) un isonitrilo,
(u) un nitro,
(v) un azido,
(w) un iminométhyle qui n'est pas substitué ou qui est substitué sur l'azote ou le carbone par un alkyle C₁₋₁₀,
(x) un oxo ou
(y) un thiono ;
R² et R₃ sont indépendamment
un hydrogène,
un alkyle C₁₋₁₀,
un aryle ; ou
R² et R3 ensembles sont un oxo ou un thiono ;
R⁴ et R⁵ sont indépendamment un hydrogène ou un carbonylalkyle ; ou
R⁴ et R⁵ sont ensembles un carbonyle ; ou
R⁴ et R¹ ensembles sont un alkylène C₁-C₃ qui n'est pas substitué ou qui est substitué par un groupement oxo ;
R⁶ et R⁷ sont tous les deux un hydrogène, ou
l'un de R⁶ et R⁷ est un hydrogène et l'autre est
un hydroxy,
un dérivé acyloxy choisi dans le groupe constitué par un formyloxy, un carbonyloxyalkyle C₁-C₁₀, un carbonyloxyaryle et un carbonyloxyaralkyle, ou
-NHR¹² dans lequel R¹² est un hydrogène, un sulfonylaryle ou un hétérosulfonylaryle qui n'est pas substitué ou qui est substitué par 1 à 3 groupements halogène ou alkyle C₁-C₃, un sulfonylalkyle ou - C(=O)-X-A-R¹³ dans lequel X est une liaison, 0 ou NH, A est une liaison ou un alkylène C₁-C₃, R¹³ est un hydrogène, un alkyle C₁-C₁₀, un aryle, un aralkyle, un hétéroaryle, un hétérocycle, ou un cycloalkyle C₃-C₇, dont n'importe quel groupement R¹³ autre qu'un hydrogène peut être substitué par un ou plusieurs groupements halogène, hydroxyle, alcoxy C₁-C₃, cyano, isonitrilo, nitro, amino, mono- ou aminodialkyle (C₁-C₃), mercapto, thioalkyle C₁-C₃, sulfinylalkyle C₁-C₃, sulfonylalkyle C₁-C₃, thioaryle, sulfinylaryle, sulfamoyle, sulfonylaryle, carboxy, carbamoyle, carbonylalkyle C₁-C₃ ou carbonylalcoxy C₁-C₃ ; ou
R⁶ et R⁷ sont ensembles un oxo, un hydroxyimino, un alcoxyimino, un aralcoxyimino ou un aminoimino ;
R⁸ est un méthyle,
un aralcoxycarbonyle, ou
un sulfonylaryle ;
R⁹ est un hydrogène,
un formyle,
un carbonylalkyle C₁₋₁₀,
un carbonylalcoxy C₁₋₁₀, ou
un arylalcoxycarbonyle ;
R¹⁰ est un hydrogène ; ou
R¹⁰ et R¹ ensembles sont un alkylène C₁-C₃ qui n'est pas substitué ou qui est substitué par un groupement oxo ;
m et n valent indépendamment zéro ou un.

5. Utilisation selon la revendication 4, dans laquelle le 8a-azalide a la formule I dans laquelle n et m valent zéro ;
R¹ est un hydrogène, un alkyle C₁₋₁₀, un alcényle C₂₋ ₁₀, un alcynyle C₂₋₁₀ ou un sulfonylaryle, dans lequel lesdits alkyle et alcényle sont facultativement substitués par un halogène, un hydroxy, un cyano, un carbonylalcoxy C₁₋₁₀, un amino, un alkylamino C₁₋₁₀, di (aminoalkyle C₁₋₁₀), un aryle ou un aralcoxycarbonyle ;
R², R³, R⁴, R⁵, R⁹ et R¹⁰ sont chacun un hydrogène ;
l'un de R⁶ et R⁷ est un hydrogène et l'autre est choisi parmi un hydroxyle, un carbonyloxyalkyle C₁₋ ₁₀, un aralkylcarbonyloxy, un amino, un amino substitué par un carbonylalkyle C₁₋₁₀, un carbonylaryle, un carbonylarylalkyle C₁₋₁₀, un carbonylalcoxy C₁₋₁₀, un carbonylarylalcoxy C₁₋₁₀, un hétéroarylcarbonyle, un carbonylhétéroarylalkyle ou un sulfonylaryle ;
R⁸ est un méthyle.

6. Utilisation selon la revendication 4, dans laquelle le 8a-azalide a la formule I dans laquelle
n et m valent zéro ;
R¹ est un méthyle, un éthyle, un propyle, un allyle, un propargyle, un 2-cyanoéthyle, un 2-hydroxyéthyle, un 3-hydroxypropyle, un 2-méthoxycarbonyléthyle, un 2-benzyloxycarbonyléthyle, un cyanométhyle, un 2-aminoéthyle, un 2-(diméthylamino) éthyle, un 2-fluoroéthyle, un 2-fluoroallyle, un benzyle ou un oxiranylméthyle ;
R², R³, R⁴, R⁵, R⁹ et R¹⁰ sont un hydrogène ;
l'un de R⁶ ou R⁷ est un hydrogène et l'autre est un hydroxy ou un amine ;
R⁸ est un méthyle.

7. Utilisation selon la revendication 6, dans laquelle R¹ est un méthyle, un éthyle, un propyle, un allyle, un 2-méthoxycarbonyléthyle ou un 2-(diméthylamino) éthyle.
